# EUROPEAN PATENT APPLICATION

(11) **EP 0 738 752 A1**
(43) Date of publication of application: **23.10.1996**
(21) Application number: 94929650.3
(22) Date of filing: 13.10.1994
(51) Int. Cl.: C08J 11/04, C12P 5/02, C12P 13/02, C12N 9/00

(54) **METHOD OF ENZYMATICALLY DEGRADING SYNTHETIC POLYMER**

(71) Applicant: KABUSHIKI KAISHA KOBE SEIKO SHO, Kobe-shi Hyogo-ken 651 (JP)
(72) Inventor: NISHIDA, Tomoaki, Shizuoka 422 (JP); DEGUCHI, Tetsuya-Kobe Corp.Res.Lab.in Kobe Steel, Hyogo 651-22 (JP); TAKAHARA, Yoshimasa-Kobe Steel Ltd., Tokyo 100 (JP)
(74) Representative: Pellmann, Hans-Bernd, Dipl.-Ing.
(86) International application number: JP9401714
(87) International publication number: WO9611972

(57) **Abstract**

A method of degrading a synthetic polymer with a mangenese-dependent peroxidase in the presence of manganese ions and further at least one additive selected from the group consisting of phosphoric, succinic and acetic acids and salts thereof. This method enables various synthetic polymers, particularly polyamides and polyolefins, to be degraded more efficiently.

## Description

### Field of the Invention

This invention relates to a process for enzymatically decomposing synthetic polymers. Such particular process contributes greatly to a satisfactory solution for treating or coping with plastic wastes that have lately created a serious social problem. Also advantageously, a decomposition product derived by the process according to the present invention can be expected to develop a new area of market.

### Background Art

Polymers resulting from synthetic preparations have found wide applications for their large extent of convenience. Even when disposed of as wastes, however, such synthetic polymers are hardly decomposable because they are totally originally alien to the natural ecosystem. This has invited great concern about environmental pollution.

Of technologies for use in bringing synthetic polymers into decomposed or otherwise degraded form, a method of decomposing a polymer of a polyamide type is known in which a microorganism (Flavobacterium sp. K172) is employed as disclosed in "Agr. Biol. Chem.", Vol. 39, No. 6, 1211-1223 (1975) and "Ferment. Eng.", Vol. 60, No. 5, 363-375 (1982).

Japanese Patent Laid-Open No. 49-55740 teaches a biologically decomposable composition made up of a synthetic resin of a thermoplastic class and rendered easily accessible to biological attack with granular starch dispersed in that resin.

The first method cited above is directed toward treating a water-soluble oligomer of nylon 6 with a molecular weight of not larger than about 2,000. This prior art method is unfeasible for the decomposition of water-insoluble high-molecular nylons, for example, of about 10,000 or above in molecular weight. This is taken to mean that such a nylon of a higher molecular weight needs to be previously made molecularly low with the aid of any suitable means.

The last-cited composition has the drawback that the starch granules can only be subjected to biological decomposition with the synthetic resin non-decomposed with the result that the composition itself simulatingly disintegrates to all appearances. In this instance, the resin itself remains in its particulate state.

In addition to the decomposition technologies discussed above, wood-rotting basidiomycetes are known as serving to decompose not only lignin and cellulose but synthetic polymers as well. As disclosed for instance in Japanese Patent Laid-Open Nos. 5-304968 and 6-70783, white rotting basidiomycetes among the wood-rotting basidiomycetes, i.e. lignin-decomposing bacteria, are known as acting to decompose synthetic polymers such as polyamides, polyolefins and the like.

As a result of further intensive researches into the foregoing wood-rotting basidiomycetes, the present invention seeks to provide a new process which enables synthetic polymers to be sufficiently effectively decomposed into a molecularly low form.

### Disclosure of the Invention

Through continued researches done by the present inventors, it has been found that synthetic polymers can be rendered readily decomposable by the action of certain specific enzymes induced from those wood-rotting basidiomycetes. Particularly noted among such enzymes is a manganese-dependent peroxydase, hereunder called Mn-P where appropriate, which is made derivable by isolation from a given microorganism.

The process according to the present invention contemplates decomposing synthetic polymers in the presence of a manganese ion with the use of Mn-P. This process obviates the need for bacteria requiring inconvenient handling and also the contamination of those materials which would become hazardous to the desired peroxydase reaction. The present invention therefore permits decomposition, with enhanced efficiency, of a variety of synthetic polymers such as nylons, polyethylenes, polyesters, polystyrenes, polyurethanes, polypropylenes and the like. Decomposing these polymers with use of Mn-P according to the present invention does not necessarily require the use of hydrogen peroxide, hence warranting commercialization with saved cost. To implement the process of the present invention, alpha-hydroxy acids are not needed which, however, are commonly accepted as absolutely necessary in the art. Conversely, such an acid has been found rather inhibitory to a peroxydase reaction intended to be achieved by the present invention.

The present invention may be carried out preferably in the presence of at least one additive selected from the group consisting of phosphoric acid, succinic acid and acetic acid, and salts thereof. This sort of additive has a role to gain facilitated decomposition of a synthetic polymer.

In accordance with the process of the present invention, satisfactory results can be produced by the use of one manganese-dependent peroxydase separated from a wood-rotting basidiomycete.

Especially noticeable results are attainable in the case of use of another manganese-dependent peroxydase separable from an NK-1148 strain.

Furthermore, conspicuous results can be obtained when still another manganese-dependent peroxydase is employed which is made derivable from the Phanerochaete chrysosporium microorganism.

In practicing the peroxydase reaction according to the present invention, the manganese ion preferably ranges in concentration from 0.1 to 10 mM.

The concentration of the phosphoric acid to be used in the reaction system is in the range of 2 to 40 mM, preferably from 3 to 30 mM, more preferably from 5 to 20 mM.

The concentration of the succinic acid to be added is in the range of 2 to 300 mM, preferably from 3 to 250 mM, more preferably from 5 to 200 mM.

The acetic acid when chosen is used in a concentration of 70 to 1,200 mM, preferably of 100 to 800 mM, more preferably of 200 to 700 mM.

The process of the present invention is especially effective in decomposing the polymers of a polyamide type or of a polyolefin type amongst the various polymers listed above.

### Best Mode for Carrying Out the Invention

From among various constituents of the NK-1148 strain that belongs to the wood-rotting basidiomycetes, the present inventors have separated and purified those components which could participate in decomposing polymers of a polyamide type. Subsequent studies of the resulting components have demonstrated that the decomposition of polymers of a polyamide type flows from the catalytic activity of Mn-P. As a consequence of similar studies on those components which could take part in the decomposition of polymers of a polyolefin type, it has been ascertained that the polymers of a polyolefin type are catalyzed and decomposed also by Mn-P.

Mn-P will now be described in detail.

Mn-P is obtainable as several different enzyme species from the wood-rotting basidiomycetes such as phanerochaete chrysosporium, moss bacteria and the like and further from the NK-1148 strain (FERM BP-1859), which is also a wood-rotting basidiomycete capable of decomposing lignin. The bacteriological properties of NK-1148 are disclosed in Japanese Patent Laid-Open No. 2-259180.

Heretofore, Mn-P has been regarded as an enzyme for use in oxidatively decomposing substances related to lignin. In light of the fact that the lignin-related substances are wholly dissimilar in chemical structure to the polymers of a polyamide type and of a polyolefin type, it was not entirely expected in the past that Mn-P would be employed to catalyze and decompose such synthetic polymers.

Upon treatment with 2,2'-azinobis(3-ethylbenzothiazoline-6-sulfonate, hereinafter referred to as ABTS), in the presence of hydrogen peroxide, MnSO4 and a salt of lactic acid, Mn-P develops a colored compound having an absorption maximum in the vicinity of 415 nm. Thus, the activity of Mn-P is determinable by absorbance measurement at an absorption band of 415 nm [Jeffrey K. Glenn and Michael H. Gold, "Arch. Biochem. Biophys.", 242, 329-341 (1985)].

Details as regards the determination of the Mn-P activity are given below.

An enzyme solution in an amount of 10 µl is incorporated in 1 ml of a reactive solution composed of a buffer solution (pH 4.5) of 20 mM succinic acid, 50 mM sodium lactate, 80 µg of ABTS and 30 mg of gelatin, followed by addition of hydrogen peroxide up to 100 µM. Reaction is run at room temperature, and increases in absorbance at 415 nm are measured. The enzymatic activity is determined by setting an absorbance increase of 0.01 during 1 minute to be one unit.

Four enzyme species, termed NK-1, NK-2, NK-3 and NK-4, have been discovered to date as Mn-P produced from the NK-1148 strain. Purification of the species is performed in the following manner.

Mn-P produced from NK-1148 is mainly secreted externally of the microorganism. When liquid culture is employed, a culture solution obtained from removal of the microorganism as by centrifugation can be purified upon being subjected to fractionation based on ammonium sulfate, gel filtration, ion exchange and the like. In the case of solid culture, an extract obtained from extraction of the resultant culture can be treated in the same manner as in the liquid culture and with use of similar means.

One preferred mode of preparation of the Mn-P species according to the present invention is illustrated below.

Into a 5-liter Erlenmeyer flask was put 1 liter of a culture medium so composed as shown in Table 1. Sterilization of the above flask was done at 120 °C for 15 minutes.

**Table 1**

| Composition of culture medium | |
|---|---|
| Glucose | 10.0g |
| KH₂PO₄ | 1.0g |
| NaH₂PO₄ | 0.2g |
| MgSO₄ · 7H₂O | 0.5g |
| MnSO₄ · 5H₂O | 48.0mg |
| Metallic salt solution | 1.0ml |
| Vitamin solution | 0.5ml |
| Ion-exchanged water | 998.5ml |

In Table 1 the metallic salt solution was prepared by dissolving, in 1 liter of distilled water, 1.5 g of nitrilotriacetic acid, 1.0 g of NaCl, 100 mg of FeSO₄ · 7H₂O, 100 mg of CoSO₄, 82 mg of CaCl₂, 100 mg of ZnSO₄, 10 mg of CuSO₄ · 5H₂O, 10 mg of AlK(SO₄)₂ and 10 mg of NaMoO₄. Moreover, the vitamin solution was prepared by dissolving, in 1 liter of distilled water, 2 mg of biotin, 2 mg of folic acid, 5 mg of thiamine hydrochloride, 5 mg of riboflavine, 10 mg of pyridine hydrochloride, 0.1 mg of cyanocobalamine, 5 mg of nicotinic acid and 5 mg of calcium pantothenic acid.

The resulting culture medium was inoculated with NK-1148, incubated by shake culture at 28 °C for 4 consecutive days.

After completion of the culture, centrifugation of the culture solution was effected to remove the microorganism therefrom. Subsequent concentration through ultrafiltration provided a crude enzyme solution.

The crude enzyme solution thus derived was thereafter fractionated and purified by means of FPLC (Fast Protein Liquid Chromatography; high-speed protein-fractionating device manufactured and supplied by Pharmacia Co.). Namely, such enzyme solution was allowed to be adsorbed by Mono Q Column (Pharmacia Co.), which had been buffered with a 10 mM-phosphoric acid buffer (pH 6.0), and then eluted by use of a NaCl-containing, 10 mM-phosphoric acid buffer (pH 6.0) and with a gradient of ionic strength set to range from 10 to 500 mM in terms of NaCl. The flow rate in such instance was 1.0 ml per minute. This elution resulted in the recovery of fraction 1 and fraction 2 each exhibiting a Mn-P activity. Subsequently, chromatofocusing using Mono PHR 5/5 Column (Pharmacia Co.) was performed. Specifically, equilibrium of the column using a 25 mM-piperazine-HCl (pH 6.0), was performed; each of the fractions was adsorbed by such column; and the fraction was eluted with the solution obtained by diluting PBE 74 (Poly Buffer 74) by ten times (adjusted in its pH to 3.0 with HCl). The flow rate here was 0.5 ml per minute. Consequently, four different enzymes, i.e., NK-1, NK-2, NK-3,and NK-4, were obtained in pure form.

The physico-chemical properties of NK-1 to NK-4 are explained below.

Molecular weights were measured by electrophoresis on an SDS-polyacrylamide gel, and each molecular weight of NK-1 to NK-4 was about 43000 ± 5000.

Isoelectric points were measured from pH values at the time of chromatofocusing. NK-1 was determined in its isoelectric point to be around pH 4.6, NK-2 around pH 4.3, NK-3 around pH 3.7 and NK-4 around pH 3.4.

As concerns absorption spectra, NK-1, NK-2 and NK-4 showed their respective absorption maxima in the neighborhood of 407 nm and NK-3 of 417 nm. Each such enzyme species was a Fe-containing enzyme.

Optimum pH ranges were determined by measuring the enzymatic activities of NK-1 to NK-4 with use of a 50 mM-acetic acid buffer (pH 3.5 to 5.0). Each of the enzymes had an optimum pH of about 4 to 4.5.

Levels of pH stability were adjudged by determining the enzymatic activity of each of NK-1 to NK-4 after having been allowed to stand at 40 °C for 10 minutes in a 20 mM-tartaric acid buffer (pH 2.5 to 3.5), a 20 mM-acetic acid buffer (pH 3.5 to 5.5) and a 20 mM-phosphoric acid buffer (pH 5.8 to 8), respectively. Each such enzyme showed a level of pH stability at from about 3.5 to 5.5.

Optimum temperatures were determined by enzymatic activity measurements of NK-1 to NK-4 at varying temperatures. Each enzyme had an optimum temperature at from about 30 to 45 °C.

Levels of temperature stability were determined by measuring the enzymatic activity of each of NK-1 to NK-4 after having been allowed to stand at each of 20 to 60 °C for 10 minutes in a 20 mM-acetic acid buffer (pH 4.5). Each such enzyme was stable at temperatures up to 45 °C.

### Experiment 1

### Effects of Mn-P on Nylon and on Polyethylene

Reactions of Mn-P with nylon and with polyethylene are explained below.

**Table 2**

| composition | reactive mixture | | | |
|---|---|---|---|---|
| | A | B | C | D |
| MnSo₄ (mM) | 0.0 | 1.0 | 0.0 | 1.0 |
| KH₂PO₄ (mM) | 0.0 | 0.0 | 10.0 | 10.0 |
| Tween 80 (wt/wt%) | 0.1 | 0.1 | 0.1 | 0.1 |
| acetic acid buffer (mM) | 40 | 40 | 40 | 40 |

To 0.5 ml of each of reactive mixtures A to D so composed as listed in Table 2 was added 1 mg of a nylon 66 membrane (nylon 66 membrane filter, Zaltrius Co.) or a polyethylene membrane (PE-1100, Asahi Chemical Co.). In the resultant mixture was then incorporated 1.5 units of each of the enzymes, NK-1 to NK-4, which had been purified as noted hereinabove.

The whole was reacted at 40°C for 48 hours.

Upon completion of the reaction, a nylon decomposition product was dissolved in hexafluoro-isopropanol (hereunder called HFIP), followed by measurement of the molecular weight of the product through gel filtration chromatography (hereunder called GPC). The GPC measurement was carried out with column, HFIP-80M (Showa Denko Co.); eluent, HFIP; flow rate, 1 ml/min; and temperature, 40°C. The solution obtained by GPC was inspected with a differential refractometer (RI). The average molecular weight was computed from a polymethyl methacrylate standard. Table 3 shows the average molecular weights of the nylon decomposition products resulting from the above treatment. The nylon prior to the treatment had a weight-average molecular weight of 85,000 and a number-average molecular weight of 50,000. Also in each of the experiments of nylon decomposition described later, a nylon before enzymatic treatment is 85,000 in weight-average molecular weight and 50,000 in number-average molecular weight as in Experiment 1.

**Table 3**

| enzyme species | reactive mixture | | | |
|---|---|---|---|---|
| | A | B | C | D |
| NK-1 ① | 81000 | 75000 | 81000 | 37000 |
| NK-1 ② | 45000 | 40000 | 46000 | 21000 |
| NK-2 ① | 82000 | 78000 | 81000 | 34000 |
| NK-2 ② | 45000 | 43000 | 44000 | 18000 |
| NK-3 ① | 83000 | 79000 | 82000 | 41000 |
| NK-3 ② | 45000 | 44000 | 44000 | 22000 |
| NK-4 ① | 82000 | 76000 | 82000 | 38000 |
| NK-4 ② | 45000 | 42000 | 44000 | 22000 |

| | | | | |
|---|---|---|---|---|
| ①... weight-average molecular weight | | | | |
| ②... number-average molecular weight | | | | |

Further, a polyethylene decomposition product after being treated as set forth above in connection with the nylon was dissolved in trichlorobenzene and measured in molecular weight by GPC under a high temperature. This GPC measurement was done with columns, Column HT Linear (Waters Inc.) and Ultrastylagel 500; eluting solution, trichlorobenzene; flow rate, 1 ml/min; and temperature, 135 . The eluate was checked by RI. The average molecular weight was computed from a polystyrene standard. In Table 4 the average molecular weights are shown of the polyethylene decomposition products treated with the enzymes according to the present invention. The polyethylene prior to the treatment had a weight-average molecular weight of 125,000 and a number-average molecular weight of 29,000. Also in each of the experiments of polyethylene decomposition described later, a polyethylene before enzymatic treatment is 125,000 in weight-average molecular weight and 29,000 in number-average molecular weight as in Experiment 1.

**Table 4**

| enzyme species | reactive mixture | | | |
|---|---|---|---|---|
| | A | B | C | D |
| NK-1 ① | 120000 | 110000 | 120000 | 50000 |
| NK-1 ② | 26000 | 23000 | 26000 | 14000 |
| NK-2 ① | 117000 | 110000 | 120000 | 46000 |
| NK-2 ② | 24000 | 23000 | 26000 | 13000 |
| NK-3 ① | 123000 | 115000 | 120000 | 60000 |
| NK-3 ② | 28000 | 25000 | 26000 | 14000 |
| NK-4 ① | 120000 | 110000 | 119000 | 49000 |
| NK-4 ② | 26000 | 23000 | 26000 | 14000 |

| | | | | |
|---|---|---|---|---|
| ①... weight-average molecular weight | | | | |
| ② ...number-average molecular weight | | | | |

As evidenced by the results of Tables 3 and 4, the nylon and polyethylene have been proved to be decomposable to a larger degree by treatment with NK-1 to NK-4. More specifically, comparison of A to B, comparison of C to D and further comparison of B to D reveal that the reactive mixtures containing MnSO₄ and KH₂PO₄ exhibit by far greater capabilities to decompose both of the test polymers.

According to the process of the present invention, synthetic polymers are rendered decomposable by catalytic reaction with a specified group of enzymes capable of taking part in the corresponding decomposition. As distinct from the case where a microorganism is employed, therefore, the process of the present invention is free from objectionable materials such as protease which would be apt to decompose the enzymes of the present invention, and substances which would be liable to block the desired decomposition reaction. Another advantage is that reaction conditions can be selected to suit a given enzyme without the need for care taken in the growing conditions of a microorganism, and hence, synthetic polymers can be decomposed with greater efficiency. For instance, owing to the optimum growth temperature of a living organism at from 28 to 37°C, conventional methods fail to gain a sufficiently high decomposition temperature. The enzymes according to the present invention ensure that temperature conditions as high as 40°C be attained in carrying out a decomposition reaction.

### Experiment 2

### Effects of Mn-P Concentrations

The effects of amounts of the enzymes (Mn-P) to be used in reactive mixtures are explained below.

Reactive Mixture D listed in Table 2 above was used as a reactive mixture and NK-4 as Mn-P. To 0.5 ml of Reactive Mixture D was added NK-4 in its three different units of 1.5, 15 and 150. The resulting mixtures were applied in decomposing a nylon under the same reaction conditions as in

### Experiment 1. Reaction limes were set for 48 hours and for 96 hours.

The average molecular weight of each of the resultant nylon decomposition products was checked by GPC and by the same procedure as in Experiment 1. The results are shown in Table 5.

**Table 5**

| enzyme concentration (unit) | weight-average molecular weight (upper row) number-average molecular weight (lower row) | |
|---|---|---|
| | reaction (48 hr) | reaction (96 hr) |
| 1.5 | 37000 | 18000 |
| | 21000 | 10000 |
| 15 | 28000 | 14000 |
| | 15000 | 7000 |
| 150 | 11000 | 5000 |
| | 5000 | 2000 |

As is clear from Table 5, the nylon is decomposable at a faster rate as the enzyme concentration is higher. Hence, increased concentration of a given enzyme leads to a rise in reaction rate. Further, the decomposition rate can be desirably selected by varying the concentration of the enzymes.

### Experiment 3

### Effects of Additives

As is generally known, the enzymatic reaction of Mn-P requires for the presence of alpha-hydroxy acids. In fact, decomposition of lignin-related substances is not feasible without addition of such acids. In decomposing synthetic polymers, however, alpha-hydroxy acids are not needed which have now been found to be rather liable to inhibit the desired decomposition reaction.

This experiment examines the effects of various acids as additives. As alpha-hydroxy acids are chosen lactic acid, malic acid, glycolic acid, citric acid and tartaric acid, while other acids are acetic acid, succinic acid and phosphoric acid.

The procedure of Experiment 3 is explained below. The concentrations of the test additives are indicated as the final concentrations.

Each of the additives was used in two concentrations of 10 mM and 50 mM and adjusted in its pH to 4.3 with NaOH. To 0.5 ml of the additive each prepared to have two varying concentrations was added MnSO₄ in an amount up to 1 mM, followed by incorporation of 1 mg of a membrane of nylon 66 and 1.5 units of NK-4. The whole was reacted at 40°C for 48 hours.

Upon completion of the reaction, the average molecular weight of each of the nylon decomposition products was inspected by GPC as in Experiment 1. The results are shown in Table 6.

Further examination was made of a reactive solution in which 10 mM of phosphoric acid was contained. From this reactive solution, markedly good results were obtained as shown in Table 6. In 10 mM of phosphoric acid containing 1 mM of MnSO₄ were incorporated lactic acid in an amount up to 50 mM and also a membrane of nylon 66 and NK-4 in the same manner as tested above. Reaction was run at 40°C for 48 hours. The average molecular weight of the nylon decomposition product was checked with the results shown also in Table 6.

**Table 6**

| additive | | weight-average molecular weight | number-average molecular weight |
|---|---|---|---|
| lactic acid | 10mM | 85000 | 50000 |
| | 50mM | 84000 | 49000 |
| malic acid | 10mM | 85000 | 49000 |
| | 50mM | 85000 | 50000 |
| glycolic acid | 10mM | 84000 | 50000 |
| | 50mM | 84000 | 49000 |
| citric acid | 10mM | 84000 | 49000 |
| | 50mM | 85000 | 49000 |
| tartaric acid | 10mM | 85000 | 50000 |
| | 50mM | 85000 | 50000 |
| acetic acid | 10mM | 75000 | 40000 |
| | 50mM | 72000 | 38000 |
| succinic acid | 10mM | 58000 | 29000 |
| | 50mM | 45000 | 25000 |
| phosphoric acid | 10mM | 38000 | 22000 |
| | 50mM | 76000 | 42000 |
| phosphoric acid 10mM + lactic acid 50mM | | 85000 | 50000 |

The results of Table 6 demonstrate that the alpha-hydroxy acids are wholly ineffective in decomposing the nylon material. The most noticeable results of decomposition were made available from the case where 10 mM of phosphoric acid was used. However, when 10 mM of such acid was employed together with lactic acid, no decomposition of the nylon took place. This means that the lactic acid acted to inhibit the desired reaction.

### Comparative Experiment 4

### Effects of Additives in Decomposing Lignin-Related Substances

As compared to Experiment 3, examination was made of the effects of various additives on the oxidative decomposition of a lignin-related substance, 2,6-dimethoxy-phenol, by the use of Mn-P. The same additives as in Experiment 3 were tested which included alpha-hydroxy acids such as lactic acid, malic acid, glycolic acid, citric acid and tartaric acid, and other acids such as acetic acid, succinic acid and phosphoric acid.

The procedure of Comparative Experiment 4 is explained below.

Each of the additives was set at the concentration listed in Table 7 and adjusted in its pH to 4.3 with NaOH. In the additive were incorporated 1 mM of MnSO₄, 2,6-dimethoxyphenol in an amount up to 1 mM and 0.2 unit of NK-4. Hydrogen peroxide was further added in an amount up to 0.1 mM. Subsequent reaction was initiated. The reactive solution was used in an amount of 0.5 ml.

2,6-Dimethoxyphenol when oxidized was increased in its absorption at 469 nm. Consequently, the absorbance at 469 nm was measured, and the increase in that absorbance was taken as the enzymatic activity. In Table 7 is listed the absorbance of the reactive solution on lapse of 5 minutes after the reaction was initiated. In Table 7, "Control" indicates the case where the enzyme NK-4 was not added.

**Table 7**

| additive | | absorbance (469nm) |
|---|---|---|
| lactic acid | 50mM | 0.6058 |
| malic acid | 50mM | 0.4544 |
| glycolic acid | 50mM | 0.5266 |
| citric acid | 50mM | 0.5020 |
| tartaric acid | 50mM | 0.5116 |
| acetic acid | 50mM | 0.0301 |
| succinic acid | 50mM | 0.0280 |
| phosphoric acid | 10mM | 0.0305 |
| control (enzyme-free) | | 0.0141 |

As is apparent from Table 7, alpha-hydroxy acids are absolutely necessary for the decomposition of the lignin-related substance by Mn-P. This denotes that the decomposition system for synthetic polymers is clearly distinguishable from that for lignin-related substances.

In Comparative Experiment 4 the omission of hydrogen peroxide as a reaction initiator showed no increase in absorbance at 469 nm. Thus, the hydrogen peroxide is also essential for the decomposition of lignin-related substances.

### Experiment 5

### Effects of Phosphoric Acid Concentrations

The effects of concentrations of phosphoric acid as an additive are explained below.

The concentrations of KH₂PO₄ were varied as shown in Table 8, while the concentration of MnSO₄ was maintained constant at 1 mM. Other reaction conditions as in Experiment 1 were followed in decomposing a nylon. The average molecular weight of the nylon decomposition product was checked by GPC as in Experiment 1. The results are shown in Table 8.

**Table 8**

| enzyme species | concentration of KH₂PO₄ (mM) | | | | |
|---|---|---|---|---|---|
| | 0 | 5 | 10 | 20 | 50 |
| NK-1 ① | 75000 | 61000 | 37000 | 47000 | 75000 |
| NK-1 ② | 40000 | 31000 | 21000 | 25000 | 40000 |
| NK-2 ① | 78000 | 55000 | 34000 | 47000 | 77000 |
| NK-2 ② | 43000 | 28000 | 18000 | 24000 | 43000 |
| NK-3 ① | 79000 | 64000 | 41000 | 50000 | 77000 |
| NK-3 ② | 44000 | 33000 | 22000 | 27000 | 43000 |
| NK-4 ① | 76000 | 63000 | 38000 | 51000 | 76000 |
| NK-4 ② | 42000 | 32000 | 22000 | 27000 | 42000 |

| | | | | | |
|---|---|---|---|---|---|
| ① ... weight-average molecular weight | | | | | |
| ② ... number-average molecular weight | | | | | |

As demonstrated by the results of Table 8, good results can be produced when KH₂PO₄ is used in concentrations of 5 mM, 10 mM, and 20 mM. The most remarkable decomposition of the nylon accrues from the case of a 10 mM-concentration.

Suitable phosphoric acid salts useful for the present invention are, in addition to KH₂PO₄, sodium phosphate, ammonium phosphate and the like.

### Experiment 6

### Effects of Succinic Acid Concentrations

The effects of concentrations of succinic acid as an additive are explained below.

The concentrations of succinic acid were varied as listed in Table 9, while the concentration of MnSO₄ was maintained constant at 1 mM. Other reaction conditions as in Experiment 1 were followed in decomposing a nylon. NK-4 was used as an enzyme. The average molecular weight of the nylon decomposition product was checked by GPC as in Experiment 1. The results are shown in Table 9.

**Table 9**

| | concentration of succinic acid (mM) | | | | | | |
|---|---|---|---|---|---|---|---|
| | 0 | 10 | 20 | 50 | 100 | 200 | 400 |
| ① | 76000 | 58000 | 50000 | 45000 | 51000 | 63000 | 76000 |
| ② | 42000 | 29000 | 38000 | 25000 | 39000 | 32000 | 42000 |

| | | | | | | | |
|---|---|---|---|---|---|---|---|
| ① ... weight-average molecular weight | | | | | | | |
| ② ... number-average molecular weight | | | | | | | |

As is clear from Table 9, the nylon is adequately decomposable at all of the test concentrations. Better results are available at concentrations of mM of 10, 20, 50, 100 and 200 and the best results at 50 mM.

### Experiment 7

### Effects of Acetic Acid Concentrations

The effects of concentrations of acetic acid as an additive are explained below.

The concentrations of acetic acid were varied as shown in Table 10, and the concentration of MnSO₄ was held at 1 mM. Other reaction conditions as in Experiment 1 were followed in decomposing a nylon. NK-4 was used as an enzyme. The average molecular weight of the nylon decomposition product was checked by GPC as in Experiment 1. The results are listed in Table 10.

**Table 10**

| | concentration of acetic acid (mM) | | | | | | |
|---|---|---|---|---|---|---|---|
| | 0 | 50 | 100 | 200 | 400 | 800 | 1000 |
| ① | 76000 | 72000 | 68000 | 58000 | 45000 | 65000 | 75000 |
| ② | 42000 | 38000 | 35000 | 29000 | 25000 | 33000 | 42000 |

| | | | | | | | |
|---|---|---|---|---|---|---|---|
| ① ... weight-average molecular weight | | | | | | | |
| ② ... number-average molecular weight | | | | | | | |

Table 10 reveals that the nylon undergoes sufficient decomposition at all of the test concentrations. Better results are derivable from concentrations of mM of 100, 200, 400 and 800 and the best results from 400 mM.

### Experiment 8

### Effects of Manganese Ion

The effects of a manganese ion in a reaction system when compared to various other metal ions are explained below.

To a 40 mM-acetic acid buffer (pH 4.3) in which 10 mM of KH₂PO₄ had been contained were added each of manganese (II), zinc (II), cobalt (II) and iron (II) as a sulfate form in such a manner as to be at 1 mM, and then 1 mg of a membrane of nylon 66 and 1.5 units of NK-4. The whole was reacted at 40°C for 48 hours. The reactive solution was used in an amount of 0.5 ml.

After completion of the reaction, the average molecular weight of the nylon decomposition product was examined by GPC as in Experiment 1. The results are listed in Table 11.

**Table 11**

| additive | weight-average molecular weight | number-average molecular weight |
|---|---|---|
| MnSO₄ 1mM | 38000 | 22000 |
| ZnSO₄ 1mM | 84000 | 49000 |
| CoSO₄ 1mM | 84000 | 49000 |
| FeSO₄ 1mM | 85000 | 49000 |

As evidenced by Table 11, the manganese ion has been proved to have an important role to decompose the nylon. The other metal ions do not serve as acceptable substitutes for the manganese ion.

### Experiment 9

### Effects of Manganese Ion Concentrations

Examination was made on the effects of concentrations of manganese ions which are added to the reactive solution.

The concentrations of MnSO₄ were varied as shown in Table 12 below, while KH₂PO₄ was held at a constant concentration of 10 mM. Other reaction conditions as in Experiment 1 were employed to decompose a nylon material. The average molecular weight of the resulting nylon decomposition product was inspected by GPC as in Experiment 1 with the results listed in Table 12.

**Table 12**

| enzyme species | concentration of MnSO₄ (mM) | | | | |
|---|---|---|---|---|---|
| | 0 | 0.1 | 1 | 10 | 100 |
| NK-1 ① | 81000 | 60000 | 37000 | 47000 | 53000 |
| NK-1 ② | 46000 | 31000 | 21000 | 25000 | 30000 |
| NK-2 ① | 81000 | 54000 | 34000 | 44000 | 50000 |
| NK-2 ② | 44000 | 27000 | 18000 | 23000 | 28000 |
| NK-3 ① | 82000 | 64000 | 41000 | 48000 | 60000 |
| NK-3 ② | 44000 | 33000 | 22000 | 26000 | 31000 |
| NK-4 ① | 82000 | 61000 | 38000 | 47000 | 54000 |
| NK-4 ② | 44000 | 31000 | 22000 | 25000 | 27000 |

| | | | | | |
|---|---|---|---|---|---|
| ① ... weight-average molecular weight | | | | | |
| ② ... number-average molecular weight | | | | | |

As is apparent from Table 12, more than 0.1 mM of MnSO₄ is highly capable of decomposing the nylon, and the case of 1 mM is most effective. Too high a concentration of MnSO₄ would be responsible for poor decomposition of the nylon. The upper limit of a manganese ion should preferably be set at 100 mM and more preferably at 10 mM from commercial standpoints.

### Experiment 10

### Effects of Various Manganese Salts

The effects of manganese salts other than the manganese sulfate employed in the preceding experiments are explained below.

To a 40 mM-acetic acid buffer (pH 4.3) containing 10 mM of KH₂PO₄ and 0.1 wt/wt % of Tween 80 were added manganese chloride or manganese acetate in concentrations shown in Table 13 below and then 1 mg of a membrane of nylon 66 and 1.5 units of NK-4. Reaction was run at 40 °C for 48 hours. The reactive solution was used in an amount of 0.5 ml.

Upon completion of the reaction, the average molecular weight of the nylon decomposition product was checked by GPC as in Experiment 1 with the results listed in Table 13.

**Table 13**

| manganese salt | weight-average molecular weight (upper row) number-average molecular weight (lower row) | | | |
|---|---|---|---|---|
| | 0.1mM | 1mM | 10mM | 100mM |
| manganese | 63000 | 38000 | 48000 | 54000 |
| chloride | 32000 | 22000 | 25000 | 27000 |
| manganese | 60000 | 35000 | 43000 | 53000 |
| acetate | 32000 | 20000 | 23000 | 27000 |

As confirmed from Table 13, no particular restriction is imposed upon the kind of magnesium salts so long as a manganese ion is added to the reaction system.

Manganese ions eligible for the present invention include, in addition to the above noted manganese sulfate, manganese chloride and manganese acetate, a variety of manganese compounds such as manganese carbonate, manganese phosphate and the like.

### Experiment 11

### Mn-P Induced From Strains Other Than NK-1148 Strain

The capability of Phanerochaete chrysosporium-induced Mn-P to decompose nylon is explained below.

By the use of Phanerochaete chrysosporium-induced Mn-P made commercially available from Tienzyme Inc. (Pennsylvania), nylon decomposition was conducted as in Experiments 5 and 9. The effects of manganese ion concentrations and of phosphate salts were examined with the results shown in Tables 14 and 15.

**Table 14**

| | concentration of MnSO₄ (mM) | | | | |
|---|---|---|---|---|---|
| | 0 | 0.1 | 1 | 10 | 100 |
| ① | 83000 | 70000 | 47000 | 53000 | 64000 |
| ② | 46000 | 38000 | 26000 | 30000 | 33000 |

| | | | | | |
|---|---|---|---|---|---|
| ① ... weight-average molecular weight | | | | | |
| ② ... number-average molecular weight | | | | | |

**Table 15**

| | concentration of KH₂PO₄ (mM) | | | | |
|---|---|---|---|---|---|
| | 0 | 5 | 10 | 20 | 50 |
| ① | 79000 | 73000 | 50000 | 61000 | 79000 |
| ② | 44000 | 40000 | 27000 | 33000 | 43000 |

| | | | | | |
|---|---|---|---|---|---|
| ① ... weight-average molecular weight | | | | | |
| ② ... number-average molecular weight | | | | | |

Mn-P derivable from Phanerochaete chrysosporium is also effective for decomposing the nylon as evident from Tables 14 and 15.

Although Mn-P produced from the NK-1148 strain was employed in Experiments 1 to 10, the enzyme is not limited in its source of origin to NK-1148 as demonstrated by Experiment 11. Mn-P derivable from various wood-rotting basidiomycetes and further from other different microorganisms are suitably useful for the practice of the present invention. Synthesized Mn-P may also be applicable.

### Experiment 12

### Effects of Hydrogen Peroxide

Generally, peroxydase type enzymes require for the presence of hydrogen peroxide as discussed hereinbefore. The effects of hydrogen peroxide on the practice of the present invention are explained below.

Two different reactive solutions were prepared with use of 1 mM of MnSO₄ and 10 mM of KH₂PO₄. One of the solutions contained hydrogen peroxide and the other devoid of such a peroxide. Both of the reactive solutions were used to enzymatically treat a nylon membrane and a polyethylene membrane under other reaction conditions as in Experiment 1. The hydrogen peroxide was added in such a manner as to be at 100 µM at the time of initiation of the reaction. After completion of the enzymatic treatment, the average molecular weights of the nylon and polyethylene decomposition products were measured as in Experiment 1. The results obtained for the nylon are shown in Table 16 and those for the polyethylene in Table 17.

**Table 16**

| enzyme species | hydrogen peroxide | |
|---|---|---|
| | not added | added |
| NK-1 ① | 37000 | 36000 |
| NK-1 ② | 21000 | 21000 |
| NK-2 ① | 34000 | 34000 |
| NK-2 ② | 18000 | 18000 |
| NK-3 ① | 41000 | 41000 |
| NK-3 ② | 22000 | 22000 |
| NK-4 ① | 38000 | 37000 |
| NK-4 ② | 22000 | 22000 |

| | | |
|---|---|---|
| ① ... weight-average molecular weight | | |
| ② ... number-average molecular weight | | |

**Table 17**

| enzyme species | hydrogen peroxide | |
|---|---|---|
| | not added | added |
| NK-1 ① | 50000 | 50000 |
| NK-1 ② | 14000 | 14000 |
| NK-2 ① | 46000 | 46000 |
| NK-2 ② | 13000 | 13000 |
| NK-3 ① | 60000 | 59000 |
| NK-3 ② | 14000 | 14000 |
| NK-4 ① | 49000 | 47000 |
| NK-4 ② | 14000 | 13000 |

| | | |
|---|---|---|
| ① ... weight-average molecular weight | | |
| ② ... number-average molecular weight | | |

In the practice of the present invention, the presence and absence of hydrogen peroxide give no appreciable difference in decomposition capability between nylon and polyethylene materials as is apparent from Tables 16 and 17. Therefore, the present invention does not always need hydrogen peroxide, bringing about economical benefits for practical purposes.

### Exploitation in Industry

As described above, the process of the present invention contributes to treating or alleviating plastic wastes that have today posed a social problem.

## Claims

1. A process for the enzymatic decomposition of synthetic polymers, comprising decomposing a synthetic polymer with the use of a manganese-dependent peroxydase and in the presence of a manganese ion.

2. The process according to claim 1 wherein the decomposition is conducted in the presence of at least one additive selected from the group consisting of phosphoric acid, succinic acid and acetic acid, and salts thereof.

3. The process according to claim 1 or 2 wherein said manganese-dependent peroxydase is separated from a wood-rotting basidiomycete.

4. The process according to claim 3 wherein said manganese-dependent peroxydase is separated from an NK-1148 strain.

5. The process according to claim 3 wherein said manganese-dependent peroxydase is separated from Phanerochaete chrysosporium.

6. The process according to claim 1 or 2 wherein said manganese ion is added in a concentration of 0.1 to 10 mM.

7. The process according to claim 1 or 2 wherein said phosphoric acid is added in a concentration of 2 to 40 mM.

8. The process according to claim 1 or 2 wherein said succinic acid is added in a concentration of 2 to 300 mM.

9. The process according to claim 1 of 2 wherein said acetic acid is added in a concentration of 70 to 1,200 mM.

10. The process according to claim 1 or 2 wherein said synthetic polymer is of a polyamide type or of a polyolefin type.
